# EUROPEAN PATENT SPECIFICATION

(11) **EP 4 223 356 B1**
(45) Date of publication and mention of the grant of the patent: **06.05.2026**
(21) Application number: 21884439.7
(22) Date of filing: 21.06.2021
(51) Int. Cl.: A61M 60/822, A61M 60/422, A61M 60/122, A61M 60/216, A61M 60/82, F04D 29/048, H02K 7/09, F16C 32/04, H02K 21/24, H02K 7/14, F04D 13/06, F04D 29/041

(54) **MAGNETIC SUSPENSION MOTOR AND MAGNETIC SUSPENSION BLOOD PUMP**
MAGNETSCHWEBEMOTOR UND MAGNETSCHWEBEBLUTPUMPE
MOTEUR ET POMPE D'ASSISTANCE CIRCULATOIRE À LÉVITATION MAGNÉTIQUE

(30) Priority: 29.10.2020 CN 202011182801
(43) Date of publication of application: 09.08.2023
(73) Proprietor: Magassist, Inc., Suzhou, Jiangsu 215000 (CN)
(72) Inventor: LOGAN, Thomas George, Suzhou, Jiangsu 215000 (CN); CLIFTON, Peter Colton James, Suzhou, Jiangsu 215000 (CN); HSU, Chiahao, Suzhou, Jiangsu 215000 (CN)
(74) Representative: Ruttensperger Lachnit Trossin Gomoll
(86) International application number: PCT/CN2021/101230
(87) International publication number: WO 2022/088698

(56) References cited:
- WO-A1-2017/033570
- WO-A1-2020/187862
- CN-A- 102 891 636
- CN-A- 107 302 294
- CN-A- 111 561 519
- CN-A- 112 546 425
- US-A1- 2002 153 790
- US-A1- 2017 302 145
- US-A1- 2019 209 752
- US-A1- 2019 356 195
- US-B1- 6 320 290
- ZHOU J ET AL: "A disk-type bearingless motor for use as satellite momentum-reaction wheel", POWER ELECTRONICS SPECIALISTS CONFERENCE; [ANNUAL POWER ELECTRONICS SPECIALISTS CONFERENCE],, vol. 4, 23 June 2002 (2002-06-23), pages 1971 - 1975, XP010596038, ISBN: 978-0-7803-7262-7

## Description

### TECHNICAL FIELD

The present disclosure generally relates to the field of medical instruments. More particularly, the present disclosure relates to a magnetic suspension motor for use in a blood pump and a magnetic suspension blood pump including the same.

### BACKGROUND ART

Heart failure is an epidemic and life-threatening disease with 1-year mortality of about 75% once deteriorating into advanced stage. In the light of limited heart donors for advanced heart failure, ventricular assist device (VAD) technology has been a viable therapeutic option to bridge the patients to transplantation or as an alternative therapy. However, adverse events (AEs) incurred by current technology still restrict these devices being used for critically ill patients. Among all, blood damage associated AEs, such as hemolysis, neurological events, stroke, and in-blood pump thrombosis, account for 20% occurrence. Hemolysis and thrombosis are primarily attributed to hyper-physiological stress and flow stagnation in rotary blood pumps (RBPs). Although hemocompatibility could be improved by hydraulic design optimization, such optimization has been extremely challenging for the RBPs with blood-immersed bearings where direct contact between rotating and stationary components is not avoidable.

Third generation VADs utilizing non-contacting bearings with hydrodynamic suspension and/or magnetic suspension technology has been developed to address the issues. Hydrodynamic bearings eliminate the direct mechanical contact between the rotor and the stator by creating a thin blood film, normally the secondary flow gap, using blood as the lubricant. The suspension force is directly related to the rotational speed and the blood film geometry between the rotating and stationary components, which changes dynamically following changes of blood pump working conditions. A strict constraint exists for hydrodynamic bearing design because of the strong interlink between the film geometry and the suspension force.

On the other hand, active magnetic bearings can keep the secondary gap constant and thus decouples the interlink between the film geometry and the suspension force. This allows more freedom for blood pump designers to optimize the flow field in the blood pump cavity as well as the flow-related hemocompatibility, which is known relevant to the shear stress, exposure time (the time interval which the blood is exposed to the shear stress), and wash-out in the secondary flow path. Therefore, magnetic suspension RBPs has gradually re-gained attention since 2015 because the newest magnetic suspension blood pump design renders a significant reduction in the size of the blood pump to be implanted in thorax and a superior performance in terms of zero occurrence of in-blood pump thrombosis.

Therefore, during the designing of magnetic suspension RBPs, it is very important to allow enough freedom in hydraulic optimization while keeping the magnetic suspension bearing performance. The critical performance indicators for magnetic suspension bearing are the numbers of the active controlled degrees-of-freedom (DoFs) and bearing stiffness in each DoF. In order to gain strong stiffness in a DoF, it is natural to control the DoF actively by electromagnetic coils. This requires to place a pair or a set of winding coils along said DoF (orientation), thus complicating the design of the secondary flow path and limiting the space for mechanical structure (for example, the secondary flow path) for the blood flow.

In addition, there are two mechanics in a magnetic suspension blood pump: (1) rotational one which is to drive the impeller; and (2) translational one which is to keep the impeller/rotor levitating. To perform these two mechanics, there are two sets of actuators in the motor to provide moving forces for the impeller/rotor. The two sets of actuators, actually two sets of winding coils or actuators in other forms, are either configured to be completely separated from each other or to be arranged more closely adjacent to each other. In either configuration, two sets of components are required, and the two sets of components need to be controlled separately (i.e., two sets of independent control circuits are required), increasing the mechanical structure complexity as well as the failure risk.

WO2017033570A1 discloses a magnetic levitation attitude control device. In order to control the attitude of a levitated rotor, a secondary coil is wound on the surface of a levitated rotor facing an electromagnetic induction stator, and the secondary coil is wound such that conductive wire parts extend from the rotation-center-axis side of the levitated rotor toward the outer circumference of the rotor, conductive wire parts extend in the circumferential direction of the levitated rotor, and conductive wire parts extend from the outer-circumferential side of the levitated rotor toward the rotation center axis.

WO2020187862A1 discloses an intravascular blood pump for percutaneous insertion into a patient's blood vessel. The blood pump comprises a pump casing having a blood flow inlet and a blood flow outlet, an impeller arranged in said pump casing so as to be rotatable about an axis of rotation. The impeller has blades sized and shaped for conveying blood from the blood flow inlet to the blood flow outlet.

US20170302145A1 discloses an electromagnetic rotary drive and a rotational device. The electromagnetic rotary drive includes a contactlessly magnetically drivable rotor that is coil-free and free of permanent magnets and that includes a magnetically effective core, and a stator by which the rotor is contactlessly magnetically drivable about a desired axis of rotation in the operating state.

Non-patent literature discloses a disk-type bearingless motor for use as satellite momentum-reaction wheel. It does not disclose a separate rotor thrust magnet and stator thrust body, and the motor disclosed is not suitable for use in blood pumps.

### SUMMARY OF THE INVENTION

One of objects of the present disclosure is to solve one or more of the above problems and realize other additional advantages.

The invention is set out in the appended set of claims.

In a first aspect of the present disclosure, a magnetic suspension motor for use in a blood pump is provided as defined in claim 1. The magnetic suspension motor comprises a stator assembly and a rotor assembly that is disposed above the stator assembly along a vertical central axis of the magnetic suspension motor, wherein a distance-adjustable axial gap is provided between the stator assembly and the rotor assembly. The stator assembly comprises a stator base, a plurality of stator teeth distributed along a circumference of the stator base and extending upward towards the gap from an upper surface of the stator base, and a stator thrust body provided in an internal cavity enclosed by the plurality of stator teeth, and the stator teeth are wound with stator coils serving as actuators. The rotor assembly comprises a rotor ring in the form of a circular ring, a rotor driving magnet disposed on a lower surface of the rotor ring, and a rotor thrust magnet disposed in an inner cavity of the rotor ring. The stator thrust body and the rotor thrust magnet are configured to generate axial magnetic lines and generate an axial repulsive force between the stator thrust body and the rotor thrust magnet. The rotor driving magnet comprises a plurality of portions, wherein each portion is magnetized along an axial direction and adjacent portions have opposite magnetization directions, so that the rotor driving magnet has a plurality of alternating magnetic poles.

According to an embodiment of the present disclosure, each portion of the rotor driving magnet has an arc shape, so that each portion is capable of being disposed on the lower surface of the rotor ring following a shape of the rotor ring.

According to an embodiment of the present disclosure, ends of the plurality of portions are abutted to each other, so that the rotor driving magnet constructed of the plurality of portions has a closed ring shape.

According to an embodiment of the present disclosure, ends of the plurality of portions are spaced apart from each other, so that spacings are presented between corresponding portions.

According to an embodiment of the present disclosure, each of the portions comprises one or more magnet segments.

According to an embodiment of the present disclosure, the rotor driving magnet comprises four portions and the stator assembly comprises eight stator teeth.

According to an embodiment of the present disclosure, the plurality of stator teeth are configured to extend substantially vertically upward from the upper surface of the stator base towards the gap.

According to an embodiment of the present disclosure, the plurality of stator teeth are configured to extend substantially spirally upward from the upper surface of the stator base towards the gap.

According to an embodiment of the present disclosure, the plurality of stator teeth are configured such that the spacing between adjacent stator teeth gradually decreases when the stator teeth extend spirally upward towards the gap.

According to an embodiment of the present disclosure, the rotor thrust magnet and the stator thrust body are both configured as solid cylindrical magnets, and a center of the rotor thrust magnet and a center of the stator thrust body are both coincident with the vertical central axis of the magnetic suspension motor in an ideal state.

According to an embodiment of the present disclosure, the rotor thrust magnet has an external diameter larger than that of the stator thrust body.

According to an embodiment of the present disclosure, the rotor thrust magnet is configured as an annular magnet with an internal cavity and the stator thrust body is configured as a solid cylindrical magnet, and a center of the rotor thrust magnet and a center of the stator thrust body are both coincident with the vertical central axis of the magnetic suspension motor in an ideal state.

According to an embodiment of the present disclosure, the rotor thrust magnet has an internal diameter of a first size and an external diameter of a second size, and the stator thrust body has an external diameter of a size between the first size and the second size of the rotor thrust magnet.

According to an embodiment of the present disclosure, the stator base is in an annular shape or a round pie shape.

According to an embodiment of the present disclosure, the stator thrust body is made of permanent magnet materials, or is formed of electromagnetic coils or electromagnets.

According to an embodiment of the present disclosure, the stator thrust body, the rotor thrust magnet and the rotor driving magnet are made of permanent magnet materials.

According to an embodiment of the present disclosure, the stator base is made of magnetic conductive materials for providing magnetically conductive connection between roots of the plurality of stator teeth.

According to an embodiment of the present disclosure, the rotor ring is made of magnetic conductive materials for providing magnetically conductive connection between corresponding portions of the rotor driving magnet.

According to an embodiment of the present disclosure, the stator thrust body is located at a position above the stator coils and close to heads of the stator teeth.

According to the present invention as claimed, the stator coil is configured to generate: a direct-axis component (D-axis component) magnetic field that is aligned with the magnetic field of each portion of the rotor driving magnet, for adjusting the axial position of the rotor assembly; and a quadrature-axis component (Q-axis component) magnetic field that is away from the magnetic field of each portion of the rotor driving magnet by half a length of one portion and thus is not aligned with the magnetic field of each portion of the rotor driving magnet, so as to drive the rotor assembly to rotate and adjust the rotational speed of the rotor assembly.

According to an embodiment of the present disclosure, each of the stator teeth is wound with a stator coil, and each of the stator coils is located between a root and a head of a corresponding stator tooth and extends a portion of a length of the corresponding stator tooth.

According to an embodiment of the present disclosure, the stator coils are interconnected in groups to form a plurality of independently controllable stator coil groups.

According to an embodiment of the present disclosure, each stator coil group is connected to an amplifier to allow currents to flow into stator coils in the stator coil group.

According to an embodiment of the present disclosure, each amplifier is independently controllable to allow currents of different magnitudes to flow into stator coils in the stator coil group that is connected to the amplifier.

According to an embodiment of the present disclosure, the magnetic suspension motor comprises a controller configured to control a value of current sent to each amplifier, so that the current in each stator coil group conforms to a preset current value for this stator coil group, thereby controlling at least one of an axial movement, a tilting movement, and a rotating movement of the rotor assembly.

According to an embodiment of the present disclosure, the controller adjusts the D-axis component magnetic field by adjusting and controlling the value of current sent to the amplifier, thereby adjusting and controlling an axial force generated on the rotor assembly to ensure that the axial gap between the rotor assembly and the stator assembly conforms to a preset distance value.

According to an embodiment of the present disclosure, the controller adjusts the Q-axis component magnetic field by adjusting and controlling the value of current sent to the amplifier, thereby adjusting and controlling a rotational torque generated on the rotor assembly to ensure that rotational speed of the rotor assembly conforms to a preset rotational speed value.

According to an embodiment of the present disclosure, the controller adjusts and controls the value of current sent to the stator coils that corresponds to two of the portions opposing each other, thereby adjusting and controlling a tilting moment generated on the rotor assembly to ensure that a tilt angle of the rotor assembly conforms to a preset tilt angle value.

According to an embodiment of the present disclosure, the magnetic suspension motor comprises one or more sensors for measuring at least one of an axial position, a tilt angle and an angular position of the rotor assembly relative to the stator assembly.

According to an embodiment of the present disclosure, the controller is configured to receive measured values from the one or more sensors and adjust the value of current sent to the stator coil group based on the measured values to implement control.

According to an embodiment of the present disclosure, the sensors are eddy current-based sensors or Hall effect sensors.

According to an embodiment of the present disclosure, a head of each stator tooth has a size larger than that of other portions of the stator tooth.

According to an embodiment of the present disclosure, the head of the stator tooth is provided with a chamfered or inclined surface, such that the head of the stator tooth is wedge-shaped.

In a second aspect of the present disclosure, a magnetic suspension blood pump is provided as defined in claim 15. The magnetic suspension blood pump comprises an impeller and a magnetic suspension motor according to the present disclosure, wherein the magnetic suspension motor is used for driving the impeller.

The additional and/or other aspects and advantages of the present disclosure will be set forth in the following description, or are obvious from the description or can be learned through the practice of the present invention.

### BRIEF DESCRIPTION OF THE DRAWINGS

With reference to the following detailed description of the specific embodiments of the present disclosure in combination with the accompanying drawings, the above-mentioned features and advantages and other features and advantages of the present disclosure as well as their implementing means will become more apparent. In the figures,
Fig. 1 is a schematic perspective view of a magnetic suspension motor according to an embodiment of the present disclosure.
Fig. 2 is a sectional view of the magnetic suspension motor of Fig. 1.
Fig. 3 is an exploded view of the magnetic suspension motor of Fig. 1.
Fig. 4 is a schematic view for a rotor driving magnet of the magnetic suspension motor of Fig. 1, in which the magnetization characteristics of the rotor driving magnet are schematically shown.
Fig. 5 is a schematic perspective view showing magnetic line loops in magnetic fields generated by a rotor thrust magnet and a stator thrust body.
Figs. 6a and 6b are schematic plan view and perspective view showing magnetic line loops in the magnetic field generated by the rotor driving magnet, respectively.
Figs. 7a and 7b are schematic plan view and perspective view showing magnetic line loops in a D-axis component magnetic field generated by the stator coils, respectively.
Figs. 8a and 8b are schematic plan view and perspective view showing magnetic line loops in a Q-axis component magnetic field generated by the stator coils, respectively.
Figs. 9a to 9c are schematic views showing axial control, tilt control and radial control for a rotor of the magnetic suspension motor, respectively.

In the drawings, like reference signs indicate like components. The examples described herein are used to illustrate exemplary aspects of the present invention, and these examples should not be construed as limiting the scope of the present disclosure in any way.

### DETAILED EMBODIMENTS

The present disclosure will be described below with reference to the drawings, in which several embodiments of the present disclosure are shown. It should be understood, however, that the present disclosure may be implemented in many different ways and is not limited to the example embodiments described below. In fact, the embodiments described hereinafter are intended to make a more complete disclosure of the present disclosure and to adequately explain the scope of the disclosure to a person skilled in the art. It should also be understood that the embodiments disclosed herein can be combined in various ways to provide many additional embodiments.

For the purpose of description, the terms "upper", "lower", "left", "right", "vertical", "horizontal", "top", "bottom", "transverse", "longitudinal" and their derivatives are all related to the orientation in the drawings of the present disclosure. However, it should be understood that the present disclosure may adopt various alternative modifications, unless otherwise clearly indicated. For example, when the apparatus in the drawings is turned over, the features previously described as being "below" other features may be described to be "above" other features at this time. The apparatus may also be otherwise oriented (rotated 90 degrees or at other orientations) and the relative spatial relationships will be correspondingly altered.

The singular forms "a/an", "the" and "said" as used in the specification, unless clearly indicated, all contain the plural forms. The words "comprising", "containing" and "including" used in the specification indicate the presence of the claimed features, but do not preclude the presence of one or more additional features. The wording "and/or" as used in the specification includes any and all combinations of one or more of the relevant items listed.

In the specification, when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being "directly on", "directly attached" to, "directly connected" to, "directly coupled" with or "directly contacting" another element, there are no intervening elements present. In the specification, references to a feature that is disposed "adjacent" another feature may have portions that overlap, overlie or underlie the adjacent feature.

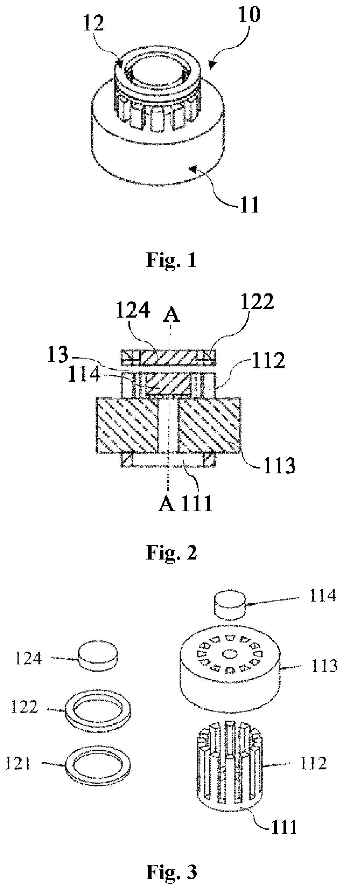

The present disclosure relates to a magnetic suspension motor. Referring to Figs. 1 to 4, a schematic structure of a magnetic suspension motor 10 according to an embodiment of the present disclosure is shown. The magnetic suspension motor 10 may include a stator assembly 11 and a rotor assembly 12 that is located above the stator assembly 11 along a vertical central axis A-A of the magnetic suspension motor 10. There may be a distance-adjustable axial gap 13 between the stator assembly 11 and the rotor assembly 12, so that the rotor assembly 12 can be suspended above the stator assembly 11 at a desired axial position.

The stator assembly 11 may include a stator base 111, and a plurality of stator teeth 112 distributed along a circumference of the stator base 111 and extending upward from an upper surface of the stator base 111 towards the gap 13. The stator base 111 may be in an annular shape or a round pie shape, for providing magnetically conductive connection between roots of the plurality of stator teeth 112. The plurality of stator teeth 112 may have a substantially same height, so that heads of the plurality of stator teeth 112 close to the rotor assembly 12 are substantially in a same plane. The plurality of stator teeth 112 may form a cylinder with an internal cavity. In an embodiment according to the present disclosure, the stator base 111 and the plurality of stator teeth 112 may be formed integrally. In another embodiment according to the present disclosure, the stator base 111 and the plurality of stator teeth 112 may be formed separately, and then the plurality of stator teeth 112 are mounted on the stator base 111 in an appropriate manner (for example, by bonding, welding, mechanical connection, etc.). The stator base 111 and the plurality of stator teeth 112 may be made of a same magnetically conductive material.

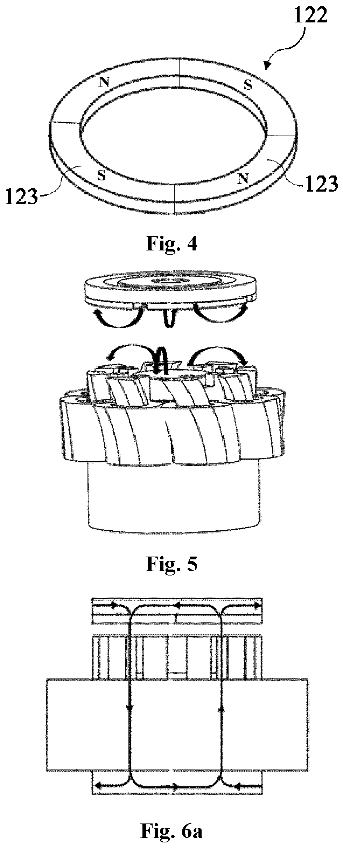

In the embodiment shown in Figs. 1 to 3, the stator teeth 112 are configured to extend substantially vertically upward from the upper surface of the stator base 111 towards the gap 13. However, the present disclosure is not limited to this. In other embodiments according to the present disclosure, the stator teeth 112 may be configured to extend substantially spirally upward from the upper surface of the stator base 111 towards the gap 13. More specifically, the stator teeth 112 may extend spirally upward from the upper surface of the stator base 111 towards the gap 13 along a same orientation on a circumferential surface of an imaginary cylinder (as shown in Figs. 5, 6b, 7b and 8b). In a preferred embodiment, a spacing between adjacent stator teeth 112 may gradually decrease when the stator teeth 112 extend spirally upward towards the gap 13. The spiral extension of the stator teeth 112 can help to increase the cross-sectional area of a surface of the stator assembly 11 facing the rotor assembly 12, thereby facilitating to increase the magnetic flux passing through the axial gap 13. In an embodiment according to the present disclosure, heads of the stator teeth 112 may have sizes larger than sizes of other portions of the stator teeth 112, so that the heads of the stator teeth 112 are in shapes of hoes or the like. In another embodiment according to the present disclosure, the heads of the stator teeth 112 may have sizes larger than sizes of other portions of the stator teeth 112, and the heads of the stator teeth 112 are provided with chamfers or inclined surfaces, thus endowing the heads of the stator teeth 112 with shapes of wedges or the like.

The stator teeth 112 may be wound with stator coils 113. The stator coils 113 function as an actuator for driving the rotor assembly 11 to perform one or more of axial movement, tilting movement, rotational movement, and the like. In an embodiment according to the present disclosure, the stator coils 113 may be distributed on the plurality of stator teeth 112 to form a distributive winding. In another embodiment according to the present disclosure, preferably, each of the stator teeth 112 is wound with a stator coil 113. Each stator coil 113 may be located between the root and the head of a corresponding stator tooth 112 and extend a portion of a length of the stator tooth 112. Each stator coil 113 may have two wiring ends. The stator coils 113 on the plurality of stator teeth 112 may be interconnected in groups to form a plurality of independently controllable stator coil groups. Each stator coil group may be connected to an amplifier, which can enable flow of currents into stator coils in the stator coil group. Each amplifier is independently controllable to allow currents of different amplitudes to flow into stator coils in the stator coil group that is in connection with this amplifier. Forming the stator coils into a plurality of independently controllable stator coil groups makes it possible to conveniently control the tilting movement and tilt angle of the rotor assembly 12, which will be described in detail below.

A stator thrust body 114 is arranged in an internal cavity of a cylinder formed by the plurality of stator teeth 112, preferably at a location above the stator coil s 113 and in proximity to the heads of the plurality of stator teeth 112. The stator thrust body 114 is configured to generate axial magnetic lines for generating axial repulsive force to suspend the rotor assembly 12 above the stator assembly 11. In an embodiment according to the present disclosure, the stator thrust body 114 is configured as a solid cylindrical magnet, with its center being coincident with a vertical central axis A-A of the magnetic suspension motor 10 in an ideal state, so that the stator thrust body 114 is arranged substantially rotationally symmetrically in the magnetic suspension motor 10. In an embodiment according to the present disclosure, the stator thrust body 114 may be made of a permanent magnet material. In another embodiment according to the present disclosure, the stator thrust body 114 may be formed of an electromagnetic coil or an electromagnet.

The rotor assembly 12 may include a rotor ring 121 in the form of an annular ring and having an internal cavity, and a rotor driving magnet 122 disposed on a lower surface of the rotor ring 121. In embodiments according to the present disclosure, the rotor driving magnet 122 is composed of a plurality of portions 123, wherein each portion has an arc shape, thus capable of being disposed on the lower surface of the rotor ring 121 following the shape of the rotor ring 121. Each portion 123 may be composed of one or more magnet segments. Each portion 123 may be magnetized along an axial direction, and adjacent portions 123 may have opposite magnetization directions, so that the rotor driving magnet 122 composed of the plurality of portions 123 has multiple alternating magnetic poles. For example, if an upper surface of a first portion is a N-pole and a lower surface thereof is a S-pole, then an upper surface of a second portion adjacent to one end of the first portion and an upper surface of a third portion adjacent to the other end of the first portion are S-poles and lower surfaces of the second and third portions are N-poles. With such a configuration, axial magnetic fields measured around the circumference of the rotor assembly 12 may contain p magnetic pole components, where p corresponds to the number of the portions 123. In embodiments according to the present disclosure, p may preferably be an even number, and p may preferably be greater than or equal to 4. When each portion 123 is composed of multiple magnet segments, the multiple magnet segments of each portion 123 may have a same magnetization direction, while adjacent portions 123 have opposite magnetization directions.

In the embodiment shown in Fig. 4, the rotor driving magnet 122 is composed of four portions 123. The ends of the four portions 123 may be abutted to each other, so that the rotor driving magnet 122 constructed of the four portions 123 has a closed ring shape. However, the present disclosure is not limited to this. The rotor driving magnet 122 may be composed of other numbers, for example, six, eight or more, of the portions 123. In addition, each portion 123 may be spaced apart from other portions 123, so that there are spacings between the portions 123 (as shown in Figs. 5, 6b, 7b and 8b). Each portion 123 may be mounted on the lower surface of the rotor ring 121 in an appropriate manner (for example, by bonding, welding, mechanical connection, etc.).

The rotor ring 121 is adapted to provide magnetically conductive connection between the portions 123 of the rotor driving magnet 122. The rotor driving magnet 122 is used to drive the rotor assembly 11 to perform one or more of axial movement, tilting movement, rotational movement and the like under the driving of the stator coil 113, which will be described in detail below. Preferably, the rotor ring 121 may have a substantially same size as the stator base 111, that is, the rotor ring 121 may have a substantially same external diameter as the stator base 111.

The rotor assembly 12 includes a rotor thrust magnet 124. The rotor thrust magnet 124 may have a substantially rotationally symmetric form, and is magnetized in an axial direction (i.e., being magnetized to generate axial magnetic lines). The rotor thrust magnet 124 may be arranged in the inner cavity of the rotor ring 121, with its center being coincident with the vertical central axis A-A of the magnetic suspension motor 10 in an ideal state.

The directions of magnetic lines of the rotor thrust magnet 124 and the stator thrust body 114 may be selected so as to generate an axial repulsive force between the rotor thrust magnet 124 and the stator thrust body 114 to suspend the rotor assembly 12 above the stator assembly 11. This can be achieved, for example, by making the direction of magnetic lines of the rotor thrust magnet 124 opposite to the direction of magnetic lines of the stator thrust body 114.

The shapes of the rotor thrust magnet 124 and the stator thrust body 114 may also be selected such that when the center of the rotor thrust magnet 124 is misaligned with the center of the stator thrust body 114, a radial component of force is generated between the rotor thrust magnet 124 and the stator thrust body 114, and this radial component of force tends to pull the rotor thrust magnet 124 and the stator thrust body 114 back to a position where their centers are aligned with each other. In the embodiment shown in Figs. 1 to 3, the rotor thrust magnet 124 is configured as a solid cylindrical magnet similar to the stator thrust body 114, and the rotor thrust magnet 124 may have an external diameter larger than that of the stator thrust body 114. In the embodiment shown in Figs. 5, 6b, 7b and 8b, the rotor thrust magnet 124 is configured as an annular magnet with an internal cavity, and has an internal diameter of a first size and an external diameter of a second size; whereas the stator thrust body 114 is configured as a solid cylindrical magnet, and has an external diameter of a size between the first size and the second size of the rotor thrust magnet 124.

In an embodiment according to the present disclosure, the number of the stator teeth 112 may be several times, such as two times, of the number of the portions 123 of the rotor driving magnet 122 (accordingly, may be several times of the number of the alternating magnetic poles). In other words, when the rotor driving magnet 122 includes four portions 123 so the rotating driving magnet 122 has four magnetic poles, the stator assembly 11 may include, for example, eight stator teeth 112.

In an embodiment according to the present disclosure, the rotor thrust magnet 124 may be made of a permanent magnet material, while the stator base 111, the stator teeth 112 and the rotor ring 121 may be made of a magnetically conductive material (e.g., metallic iron, etc.).

Next, the operation principle and control of the magnetic suspension motor 10 according to the present disclosure will be described with reference to Figs. 5 to 9c.

Fig. 5 shows magnetic line loops in a magnetic field between the stator thrust body 114 and the rotor thrust magnet 124. As can be clearly seen from Fig. 5, the magnetic lines of the stator thrust body 114 and those of the rotor thrust magnet 124 flow oppositely at positions facing each other, thereby generating an axial repulsive force between the stator thrust body 114 and the rotor thrust magnet 124 and further generating an upward axial force on the rotor ring 121 of the rotor assembly 12, which enables the whole rotor assembly 12 to be suspended above the stator assembly 11. When the rotor assembly 12 is brought to be closer to the stator assembly 11, the magnetic lines may be pushed towards the radial direction more intensively, and such distortion of the magnetic field may change the axial force.

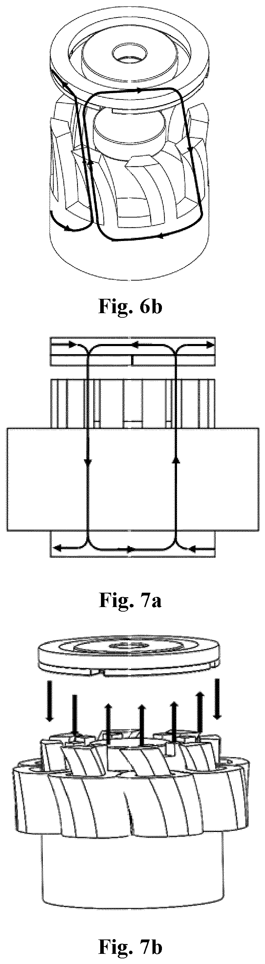

Figs. 6a and 6b show a magnetic line loop in a magnetic field generated by one portion 123 of the rotor driving magnet 122 of the rotor assembly 12 through a schematic plan view and a schematic perspective view, respectively. As shown in Figs. 6a and 6b, the magnetic line of said portion 123 can go downward through the portion 123 itself, further downward through the stator tooth 112 to reach the stator base 111, then through a part of the stator base 111 along a circumference of the stator base 111 (when the rotor driving magnet 122 includes four portions 123, the magnetic line goes around the circumference of the stator base 111 by 90 degrees to be through a quarter of the circumference of the stator base 111), then upward through another stator tooth 112, further upward through the body of another portion 123, which is adjacent to the portion 123 and has an opposite magnetization direction, to reach the rotor ring 121, and then through a part of the rotor ring 121 along a circumference of the rotor ring 121 in a direction opposite to the direction in which the magnetic lines pass through the stator base 111, so as to form a complete magnetic field loop. The portions 123 with the same magnetization direction can generate magnetic line loops with opposite magnetic line flow directions. Therefore, the whole rotor driving magnet 122 can generate magnetic line loops in which the flow directions of the magnetic lines alternately change.

The magnetic field generated by the rotor driving magnet 122 may interact with the magnetic field generated by the stator coil 113 to generate corresponding magnetic axial component and magnetic torque component, thereby generating an axial force for suspending the rotor assembly 12 above the stator assembly 11 and a rotational torque for rotating the rotor assembly 12.

Specifically, the magnetic field generated by the stator coil 113 on the stator tooth 112 can be considered as having two component magnetic fields: a direct-axis component (D-axis component) magnetic field, the magnetic line loop of which is aligned with the magnetic line loop of the magnetic field generated by the magnetic poles (i.e., the portions 123) of the rotor driving magnet 122; and a quadrature-axis component (Q-axis component) magnetic field, the magnetic line loop of which is away from the magnetic line loop of the magnetic field generated by the magnetic poles (i. e., the portions 123) of the rotor driving magnet 122 by half a length of one portion 123 and thus not aligned with the magnetic line loop of the magnetic field generated by the magnetic poles.

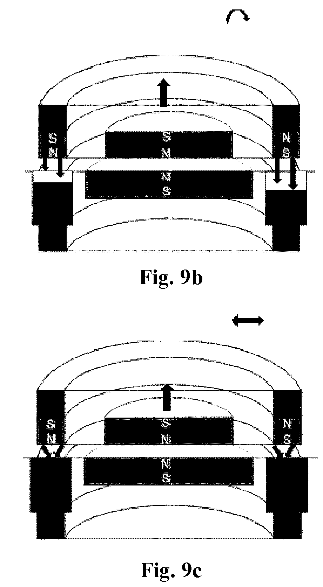

Figs. 7a and 7b show a magnetic line loop in the D-axis component magnetic field generated by the stator coil 113 through a schematic plan view and a perspective view, respectively. As can be seen, the magnetic line loop of the D-axis component magnetic field is completely aligned with the magnetic line loop generated by the rotor driving magnet 122, and the flow direction of the magnetic lines in the D-axis component magnetic field can be the same as or opposite to the flow direction of the magnetic lines generated by the rotor driving magnet 122, so that the net magnetic field below the rotor driving magnet 122 can be intensified or weakened. The D-axis component magnetic field generated by the stator coil 113 may not generate a rotational torque on the rotor assembly 12 to rotate the rotor assembly 12, but it can generate a downward attractive force between the rotor assembly 12 and the stator assembly 11, which attractive force may tend to attract the rotor assembly 12 towards the stator assembly 11 to close the gap 13 and which attractive force is proportional to the square of intensity of the net magnetic field. By use of such property, the net axial force acting on the rotor assembly 12 can be adjusted by changing the magnitude of the D-axis component magnetic field generated by the stator coil 113. As this net axial force compensates the upward axial force generated by the stator thrust body 114 and the rotor thrust magnet 124, the axial position of the rotor assembly 12 can be controlled (see Fig. 9a).

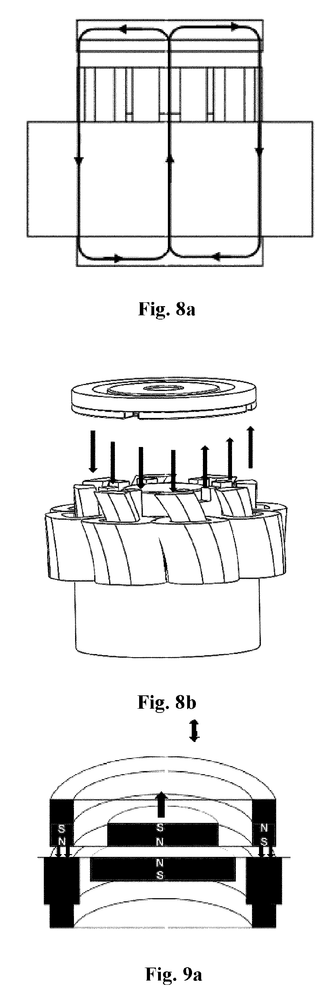

Figs. 8a and 8b show a magnetic line loop in the Q-axis component magnetic field generated by the stator coil 113 through a schematic plan view and a schematic perspective view, respectively. It can be seen that the magnetic line loop of the Q-axis component magnetic field is away from the magnetic line loop generated by the rotor driving magnet 122 by half a length of one portion 123 and thus is not aligned with the magnetic line loop generated by the rotor driving magnet 122. Since the magnetic line loop of the Q-axis component magnetic field generated by the stator coil 113 is not aligned with the magnetic line loop generated by the rotor driving magnet 122, a rotational force may be generated therebetween to try to align them, and this rotational force can generate a rotating moment on the rotor assembly 12 to rotate the rotor assembly 12.

The magnetic suspension motor 10 according to the present disclosure is operated and controlled based on the above principles. However, unlike the conventional motors, in the magnetic suspension motor 10 according to the present disclosure, the D-axis component magnetic field for each magnetic pole (i.e., each portion 123) can be independently controlled (by means of the independently controllable stator coil group as described above), thereby allowing the axial force on different sides of the rotor assembly 12 of the magnetic suspension motor 10 to be controlled to different values. This allows a tilting torque to be applied to the rotor assembly 12, thereby controlling the tilt angle of the rotor assembly 12 (see Fig. 9b). In addition, the stability of the rotor assembly 12 of the magnetic suspension motor 10 according to the present disclosure is passively obtained. When the center of the rotor assembly 12 deviates from the vertical central axis A-A of the magnetic suspension motor 10, a radial component of force tending to align them with each other will be generated between the stator base 111 and the rotor ring 121, and this radial component of force pulls the rotor assembly 12 in a radial direction back to a position where it is aligned with the stator assembly 11 (see Fig. 9c).

The magnetic suspension motor 10 according to the present disclosure may include a controller configured to control at least one of an axial movement, a tilting movement, and a rotational movement of the rotor assembly 12. The controller may control the value of current sent to each stator coil group by independently controlling each amplifier with a manner to control voltage or current, so that the current in each stator coil group conforms to a preset current value for said stator coil group, said preset current value being adjustable, so as to generate a net magnetomotive force from the stator assembly 11. In an embodiment according to the present disclosure, by adjusting and controlling the value of current sent to the stator coil group, the controller can adjust the D-axis component magnetic field and the Q-axis component magnetic field of the stator coil 113 corresponding to p magnetic poles so as to adjust and control the rotational torque and the axial force generated on the rotor assembly 12, wherein the controller adjusts and controls the axial force generated on the rotor assembly 12 to ensure that the axial gap between the rotor assembly 12 and the stator assembly 11 conforms to a preset distance value, and the controller adjusts and controls the rotational torque generated on the rotor assembly 12 to ensure that the rotational speed of the rotor assembly 12 conforms to a preset rotational speed value. In another embodiment according to the present disclosure, the controller can adjust and control the values of currents sent to two stator coils 113 that correspond to two opposite magnetic poles (the portions 213) by independently controlling corresponding amplifiers with a manner to control voltage or current, thereby adjusting and controlling the tilting moment generated on the rotor assembly 12 to ensure that the tilt angle of the rotor assembly 12 conforms to a preset tilt angle value.

The magnetic suspension motor 10 according to the present disclosure may further include one or more sensors for measuring at least one of the axial position (height), the tilt angle, and the angular position of the rotor assembly 12 relative to the stator assembly 11. The controller may receive measured values from the one or more sensors and adjust the value of current sent to the corresponding stator coils or stator coil groups based on the measured values to implement control. In the embodiments according to the present disclosure, the one or more sensors may be any one or more of eddy current-based sensors, Hall effect sensors, and other suitable types of sensors.

The magnetic suspension motor 10 according to the present disclosure may be used, for example, in an in-vivo or in-vitro magnetic suspension blood pump for driving an impeller of the in-vivo or in-vitro magnetic suspension blood pump.

The magnetic suspension motor 10 according to the present disclosure only requires one set of independently controllable stator coils 113 to simultaneously generate rotation driving force and axial translation driving force, avoiding the need for using two sets of different stator coils to generate the rotation driving force and the axial translation driving force respectively, like the motors in the prior art, thus effectively reducing the number of elements of the magnetic suspension motor 10 and accordingly decreasing the size and the risk of failure of the magnetic suspension motor 10. On this basis, the magnetic suspension motor 10 according to the present disclosure allows the stator coils 113 that is served as actuators to be placed merely on one side of the rotor assembly 12 (e.g., below the rotor assembly 12), so that when it is applied to a magnetic suspension blood pump, the flow path design of the blood pump can have more degrees-of-freedom and flexibility, and thus the flow path of the blood pump can have as simple a structure as possible. In this way, the fluid dynamics of the magnetic suspension blood pump becomes very simple, thus minimizing the damage to blood.

Furthermore, for the magnetic suspension motor 10 according to the present disclosure, the axial position, the tilting position, and the rotational speed of the rotor assembly 12 can be adjusted as required by adjusting the magnitude of the current sent to different stator coil groups by use of a single controller, so multi-degree-of-freedom control of the magnetic suspension motor 10 can be realized by a single controller, thus realizing better control through simple mechanical and control mechanisms.

## Claims

1. A magnetic suspension motor (10) for use in a blood pump, comprising a stator assembly (11) and a rotor assembly (12) that is disposed above the stator assembly along a vertical central axis (A-A) of the magnetic suspension motor (10), wherein a distance-adjustable axial gap (13) is provided between the stator assembly (11) and the rotor assembly (12);
**characterized in that** the stator assembly (11) comprises a stator base (111), a plurality of stator teeth (112) distributed along a circumference of the stator base (111) and extending upward towards the gap (13) from an upper surface of the stator base (111), and a stator thrust body (114) provided in an internal cavity enclosed by the plurality of stator teeth (112), and the stator teeth are wound with stator coils (113) serving as actuators;
wherein the rotor assembly (12) comprises a rotor ring (121) in the form of a circular ring, a rotor driving magnet (122) disposed on a lower surface of the rotor ring (121), and a rotor thrust magnet (124) disposed in an inner cavity of the rotor ring (121);
wherein the stator thrust body (114) and the rotor thrust magnet (124) are configured to generate axial magnetic lines and generate an axial repulsive force between the stator thrust body (114) and the rotor thrust magnet (124);
wherein the rotor driving magnet (122) comprises a plurality of portions (123), each portion being magnetized along an axial direction and adjacent portions having opposite magnetization directions, so that the rotor driving magnet (122) has a plurality of alternating magnetic poles; and
wherein the stator coils (113) are configured to generate: a direct-axis component magnetic field that is aligned with a magnetic field of each portion (123) of the rotor driving magnet (122), for adjusting an axial position of the rotor assembly (12); and a quadrature-axis component magnetic field that is away from the magnetic field of each portion (123) of the rotor driving magnet (122) by half a length of one portion and thus is not aligned with the magnetic field of each portion (123)of the rotor driving magnet (122), for driving the rotor assembly (12) to rotate and adjusting a rotational speed of the rotor assembly (12).

2. The magnetic suspension motor (10) according to claim 1, **characterized in that** each portion (123) of the rotor driving magnet (122) has an arc shape, so that each portion (123) is capable of being disposed on the lower surface of the rotor ring (121) following a shape of the rotor ring (121); preferably, the rotor driving magnet (122) comprises four portions (123) and the stator assembly (11) comprises eight stator teeth (112); and preferably, each of the portions (123) comprises one or more magnet segments.

3. The magnetic suspension motor (10) according to claim 2, **characterized in that** ends of the plurality of portions (123) are abutted to each other, so that the rotor driving magnet (122) constructed of the plurality of portions (123) has a closed ring shape; or
wherein ends of the plurality of portions (123) are spaced apart from each other, so that spacings are presented between corresponding portions.

4. The magnetic suspension motor (10) according to claim 1, **characterized in that** the plurality of stator teeth (112) are configured to extend substantially vertically or substantially spirally upward from the upper surface of the stator base (111) towards the gap (13), preferably, the plurality of stator teeth (112) are configured such that a spacing between adjacent stator teeth gradually decreases when the stator teeth (112) extend substantially spirally upward towards the gap (13).

5. The magnetic suspension motor (10) according to claim 1, **characterized in that** the rotor thrust magnet (124) and the stator thrust body (114) are both configured as solid cylindrical magnets, and wherein a center of the rotor thrust magnet (124) and a center of the stator thrust body (114) are both coincident with the vertical central axis (A-A) of the magnetic suspension motor (10) in an ideal state, preferably, the rotor thrust magnet (124) has an external diameter larger than that of the stator thrust body (114).

6. The magnetic suspension motor (10) according to claim 1, **characterized in that** the rotor thrust magnet (124) is configured as an annular magnet with an internal cavity and the stator thrust body (114) is configured as a solid cylindrical magnet, and wherein a center of the rotor thrust magnet (124) and a center of the stator thrust body (114) are both coincident with the vertical central axis (A-A) of the magnetic suspension motor (10) in an ideal state, preferably, the rotor thrust magnet (124) has an internal diameter of a first size and an external diameter of a second size, and the stator thrust body (114) has an external diameter of a size between the first size and the second size of the rotor thrust magnet (124).

7. The magnetic suspension motor (10) according to claim 1, **characterized in that** the stator base (111) is in an annular shape or a round pie shape.

8. The magnetic suspension motor (10) according to claim 1, **characterized in that** the stator thrust body (114) is located at a position above the stator coils (113) and close to heads of the stator teeth (112), preferably, the stator thrust body (114) is made of permanent magnet materials or is formed of electromagnetic coils or electromagnets, and preferably the rotor thrust magnet (124) and the rotor driving magnet (122) are both made of permanent magnet materials.

9. The magnetic suspension motor (10) according to claim 1, **characterized in that** the stator base (111) is made of magnetic conductive materials for providing magnetically conductive connection between roots of the plurality of stator teeth (112); and/or
wherein the rotor ring is made of magnetic conductive materials for providing magnetically conductive connection between corresponding portions of the rotor driving magnet (122).

10. The magnetic suspension motor (10) according to claim 1, **characterized in that** each of the stator teeth (112) is wound with a stator coil (113), and each of the stator coils (113) is located between a root and a head of a corresponding stator tooth (112) and extends a portion of a length of the corresponding stator tooth (112), preferably, the stator coils (113) are interconnected in groups to form a plurality of independently controllable stator coil groups, preferably, each stator coil group is connected to an amplifier to allow currents to flow into stator coils (113) in the stator coil group, and preferably, each amplifier is independently controllable to allow currents of different magnitudes to flow into stator coils (113) in the stator coil group that is connected to the amplifier.

11. The magnetic suspension motor (10) according to claim 10, **characterized in that** the magnetic suspension motor (10) comprises a controller configured to control a value of current sent to each amplifier, so that the current in each stator coil group conforms to a preset current value for this stator coil group, thereby controlling at least one of an axial movement, a tilting movement, and a rotating movement of the rotor assembly (12).

12. The magnetic suspension motor (10) according to claim 11, **characterized in that** the controller is configured to implement at least one of following operations:
i) the controller adjusts the direct-axis component magnetic field by adjusting and controlling the value of current sent to the amplifier, thereby adjusting and controlling an axial force generated on the rotor assembly (12) to ensure that the axial gap (13) between the rotor assembly (12) and the stator assembly (11) conforms to a preset distance value;
ii) the controller adjusts the quadrature-axis component magnetic field by adjusting and controlling the value of current sent to the amplifier, thereby adjusting and controlling a rotational torque generated on the rotor assembly (12) to ensure that the rotational speed of the rotor assembly (12) conforms to a preset rotational speed value; and
iii) the controller adjusts and controls the value of current sent to the stator coils (113) that corresponds to two of the portions opposing each other, thereby adjusting and controlling a tilting moment generated on the rotor assembly (12) to ensure that a tilt angle of the rotor assembly (12) conforms to a preset tilt angle value.

13. The magnetic suspension motor (10) according to claim 11, **characterized in that** the magnetic suspension motor (10) comprises one or more sensors for measuring at least one of the axial position, a tilt angle and an angular position of the rotor assembly (12) relative to the stator assembly (11), preferably, the controller is configured to receive measured values from the one or more sensors and adjust the value of current sent to the stator coil group based on the measured values to implement control, and preferably, the sensors are eddy current-based sensors or Hall effect sensors.

14. The magnetic suspension motor (10) according to claim 1, **characterized in that** a head of each stator tooth (112) has a size larger than that of other portions of the stator tooth (112), preferably, the head of the stator tooth (112) is provided with a chamfered or inclined surface, such that the head of the stator tooth (112) is wedge-shaped.

15. A magnetic suspension blood pump, **characterized in that** the magnetic suspension blood pump comprises an impeller and a magnetic suspension motor (10) according to any one of claims 1 to 14, wherein the magnetic suspension motor (10) is used for driving the impeller.

## Patentansprüche

1. Magnetschwebemotor (10) zur Verwendung in einer Blutpumpe, umfassend eine Statoranordnung (11) und eine Rotoranordnung (12), die überhalb der Statoranordnung entlang einer vertikalen Mittenachse (A-A) des Magnetschwebemotors (10) angeordnet ist, wobei zwischen der Statoranordnung (11) und der Rotoranordnung (12) ein Spalt (13) mit einstellbarem Abstand vorgesehen ist;
**dadurch gekennzeichnet, dass** die Statoranordnung (11) eine Statorbasis (111) umfasst, eine Mehrzahl von Statorzähnen (112), die entlang eines Umfangs der Statorbasis (111) verteilt sind und sich von einer Oberseite der Statorbasis (111) nach oben in Richtung des Spalts (13) erstrecken, sowie einen Statorschubkörper (114), der in einem von der Mehrzahl von Statorzähnen (112) umschlossenen Innenhohlraum vorgesehen ist, und wobei die Statorzähne mit Statorspulen (113) umwickelt sind, die als Aktuatoren dienen;
wobei die Rotoranordnung (12) einen Rotorring (121) in Form eines kreisförmigen Rings, einen an einer Unterseite des Rotorrings (121) angeordneten Rotorantriebsmagneten (122) und einen in einem inneren Hohlraum des Rotorrings (121) angeordneten Rotorschubmagneten (124) umfasst;
wobei der Statorschubkörper (114) und der Rotorschubmagnet (124) dazu ausgebildet sind, axiale Magnetfeldlinien zu erzeugen und eine axiale Abstoßungskraft zwischen dem Statorschubkörper (114) und dem Rotorschubmagneten (124) zu erzeugen;
wobei der Rotorantriebsmagnet (122) eine Mehrzahl von Abschnitten (123) umfasst, wobei jeder Abschnitt entlang einer axialen Richtung magnetisiert ist und wobei benachbarte Abschnitte entgegengesetzte Magnetisierungsrichtungen aufweisen, so dass der Rotorantriebsmagnet (122) eine Mehrzahl von abwechselnden Magnetpolen aufweist; und wobei die Statorspulen (113) dazu ausgebildet sind, Folgendes zu erzeugen: ein Magnetfeld mit einer Direktachsenkomponente, das mit dem Magnetfeld jedes Abschnitts (123) des Rotorantriebsmagneten (122) ausgerichtet ist, um eine axiale Position der Rotoranordnung (12) einzustellen; und ein Magnetfeld mit einer Quadraturachsenkomponente, das um eine halbe Länge eines Abschnitts vom Magnetfeld jedes Abschnitts (123) des Rotorantriebsmagneten (122) entfernt ist und somit nicht mit dem Magnetfeld jedes Abschnitts (123) des Rotorantriebsmagneten (122) ausgerichtet ist, um die Rotoranordnung (12) zur Drehung anzutreiben und eine Drehzahl der Rotoranordnung (12) einzustellen.

2. Magnetschwebemotor (10) nach Anspruch 1, **dadurch gekennzeichnet, dass** jeder Abschnitt (123) des Rotorantriebsmagneten (122) eine Bogenform aufweist, so dass jeder Abschnitt (123) in der Lage ist, an der Unterseite des Rotorrings (121) entsprechend der Form des Rotorrings (121) angeordnet zu sein; bevorzugt wobei der Rotorantriebsmagnet (122) vier Abschnitte (123) aufweist und die Statoranordnung (11) acht Statorzähne (112) aufweist; und bevorzugt wobei jeder der Abschnitte (123) ein oder mehrere Magnetsegmente aufweist.

3. Magnetschwebemotor (10) nach Anspruch 2, **dadurch gekennzeichnet, dass** die Enden der Mehrzahl von Abschnitten (123) aneinander anliegen, so dass der aus der Mehrzahl von Abschnitten (123) gebildete Rotorantriebsmagnet (122) eine geschlossene Ringform aufweist; oder
wobei die Enden der Mehrzahl von Abschnitten (123) zueinander beabstandet sind, so dass zwischen entsprechenden Abschnitten Abstände vorhanden sind.

4. Magnetschwebemotor (10) nach Anspruch 1, **dadurch gekennzeichnet, dass** die Mehrzahl von Statorzähnen (112) dazu ausgebildet sind, sich im Wesentlichen vertikal oder im Wesentlichen spiralförmig von der Oberseite der Statorbasis (111) in Richtung des Spalts (13) zu erstrecken; bevorzugt wobei die Mehrzahl von Statorzähnen (112) so ausgebildet ist, dass ein Abstand zwischen benachbarten Statorzähnen allmählich abnimmt, wenn sich die Statorzähne (112) im Wesentlichen spiralförmig nach oben in Richtung des Spalts (13) erstrecken.

5. Magnetschwebemotor (10) nach Anspruch 1, **dadurch gekennzeichnet, dass** der Rotorschubmagnet (124) und der Statorschubkörper (114) beide als massive zylindrische Magnete ausgebildet sind, und wobei im Idealzustand ein Mittelpunkt des Rotorschubmagneten (124) und ein Mittelpunkt des Statorschubkörpers (114) mit der vertikalen Mittenachse (A-A) des Magnetschwebemotors (10) zusammenfallen, bevorzugt wobei der Rotorschubmagnet (124) einen größeren Außendurchmesser aufweist als der Statorschubkörper (114).

6. Magnetschwebemotor (10) nach Anspruch 1, **dadurch gekennzeichnet, dass** der Rotorschubmagnet (124) als ringförmiger Magnet mit einem inneren Hohlraum ausgebildet ist und der Statorschubkörper (114) als massiver zylindrischer Magnet ausgebildet ist, und wobei ein Mittelpunkt des Rotorschubmagneten (124) und ein Mittelpunkt des Statorschubkörpers (114) im Idealzustand beide mit der vertikalen Mittenachse (A-A) des Magnetschwebemotors (10) zusammenfallen, bevorzugt wobei der Rotorschubmagnet (124) einen Innendurchmesser einer ersten Größe und einen Außendurchmesser einer zweiten Größe aufweist und der Statorschubkörper (114) einen Außendurchmesser einer Größe zwischen der ersten Größe und der zweiten Größe des Rotorschubmagneten (124) aufweist.

7. Magnetschwebemotor (10) nach Anspruch 1, **dadurch gekennzeichnet, dass** die Statorbasis (111) eine ringförmige oder eine runde, tortenförmige Gestalt aufweist.

8. Magnetschwebemotor (10) nach Anspruch 1, **dadurch gekennzeichnet, dass** sich der Statorschubkörper (114) an einer Position oberhalb der Statorspulen (113) und nahe den Spitzen der Statorzähne (112) befindet, bevorzugt wobei der Statorschubkörper (114) aus Permanentmagnetmaterialien gefertigt oder aus elektromagnetischen Spulen oder Elektromagneten gebildet ist, und bevorzugt der Rotorschubmagnet (124) und der Rotorantriebsmagnet (122) beide aus Permanentmagnetmaterialien gefertigt sind.

9. Magnetschwebemotor (10) nach Anspruch 1, **dadurch gekennzeichnet, dass** die Statorbasis (111) aus magnetisch leitfähigen Materialien gefertigt ist, um eine magnetisch leitfähige Verbindung zwischen den Wurzeln der Mehrzahl von Statorzähnen (112) herzustellen; und/oder
wobei der Rotorring aus magnetisch leitfähigen Materialien gefertigt ist, um eine magnetisch leitfähige Verbindung zwischen entsprechenden Abschnitten des Rotorantriebsmagneten (122) herzustellen.

10. Magnetschwebemotor (10) nach Anspruch 1, **dadurch gekennzeichnet, dass** jeder der Statorzähne (112) mit einer Statorspule (113) gewickelt ist, und jede der Statorspulen (113) zwischen einer Wurzel und einer Spitze eines entsprechenden Statorzahns (112) angeordnet ist und sich über einen Teil der Länge des entsprechenden Statorzahns (112) erstreckt, bevorzugt wobei die Statorspulen (113) in Gruppen miteinander verbunden sind, um eine Mehrzahl von unabhängig steuerbaren Statorspulgruppen zu bilden, bevorzugt wobei jede Statorspulengruppe mit einem Verstärker verbunden ist, um den Stromfluss in die Statorspulen (113) der Statorspulengruppe zu ermöglichen, und bevorzugt wobei jeder Verstärker unabhängig steuerbar ist, um den Stromfluss mit unterschiedlichen Stromstärken in die Statorspulen (113) der Statorspulengruppe zu ermöglichen, die mit dem Verstärker verbunden ist.

11. Magnetschwebemotor (10) nach Anspruch 10, **dadurch gekennzeichnet, dass** der Magnetschwebemotor (10) eine Steuerung umfasst, die dazu ausgebildet ist, einen Wert des an jeden Verstärker gesendeten Stroms derart zu steuern, dass der Strom in jeder Statorspulengruppe einem voreingestellten Stromwert für diese Statorspulengruppe entspricht, wodurch wenigstens eine der folgenden Bewegungen der Rotoranordnung gesteuert wird: eine axiale Bewegung, eine Kippbewegung und eine Drehbewegung der Rotoranordnung (12).

12. Magnetschwebemotor (10) nach Anspruch 11, **dadurch gekennzeichnet, dass** die Steuerung dazu ausgebildet ist, wenigstens einen der folgenden Vorgänge auszuführen:
i) die Steuerung stellt das Magnetfeld der Direktachsenkomponente ein indem sie den Wert des an den Verstärker gesendeten Stroms einstellt und steuert, wodurch eine an der Rotoranordnung (12) erzeugte Axialkraft eingestellt und gesteuert wird, um sicherzustellen, dass der Axialspalt (13) zwischen der Rotoranordnung (12) und der Statoranordnung (11) einem voreingestellten Abstandswert entspricht;
ii) die Steuerung stellt das Magnetfeld der Quadraturachsenkomponente ein indem sie den Wert des an den Verstärker gesendeten Stroms einstellt und steuert, wodurch ein an der Rotoranordnung (12) erzeugtes Drehmoment eingestellt und gesteuert wird, um sicherzustellen, dass die Drehzahl der Rotoranordnung (12) einem voreingestellten Drehzahlwert entspricht; und
iii) die Steuerung stellt ein und steuert den Wert des an die Statorspulen (113) gesendeten Stroms, der zwei der einander gegenüberliegenden Abschnitte entspricht, wodurch ein an der Rotoranordnung (12) erzeugtes Kippmoment eingestellt und gesteuert wird, um sicherzustellen, dass ein Kippwinkel der Rotoranordnung (12) einem voreingestellten Kippwinkelwert entspricht.

13. Magnetschwebemotor (10) nach Anspruch 11, **dadurch gekennzeichnet, dass** der Magnetschwebemotor (10) einen oder mehrere Sensoren zum Messen wenigstens einer der folgenden Größen umfasst: der axialen Position, eines Kippwinkels und einer Winkelposition der Rotoranordnung (12) relativ zur Statoranordnung (11), bevorzugt wobei die Steuerung dazu ausgebildet ist, Messwerte von dem einen oder den mehreren Sensoren zu empfangen und den Wert des an die Statorspulengruppe gesendeten Stroms auf der Grundlage der Messwerte einzustellen, um eine Steuerung durchzuführen, und bevorzugt wobei die Sensoren Wirbelstromsensoren oder Hall-Effekt-Sensoren sind.

14. Magnetschwebemotor (10) nach Anspruch 1, **dadurch gekennzeichnet, dass** ein Kopf jedes Statorzahns (112) eine größere Größe aufweist als andere Abschnitte des Statorzahns (112), bevorzugt wobei der Kopf des Statorzahns (112) mit einer abgeschrägten oder geneigten Fläche versehen ist, so dass der Kopf des Statorzahns (112) keilförmig ist.

15. Eine magnetisch gelagerte Blutpumpe, **dadurch gekennzeichnet, dass** die magnetisch gelagerte Blutpumpe ein Laufrad und einen Magnetschwebemotor (10) gemäß einem der Ansprüche 1 bis 14 umfasst, wobei der Magnetschwebemotor (10) zum Antreiben des Laufrads verwendet wird.

## Revendications

1. Un moteur à suspension magnétique (10) destiné à être utilisé dans une pompe à sang, comprenant un ensemble stator (11) et un ensemble rotor (12) qui est disposé au-dessus de l'ensemble stator le long d'un axe central vertical (A-A) du moteur à suspension magnétique (10), dans lequel un entrefer axial (13) à distance réglable est prévu entre l'ensemble stator (11) et l'ensemble rotor (12) ;
**caractérisé en ce que** l'ensemble stator (11) comprend une base de stator (111), une pluralité de dents de stator (112) réparties le long d'une circonférence de la base de stator (111) et s'étendant vers le haut en direction de l'entrefer (13) à partir d'une surface supérieure de la base de stator (111), et un corps de poussée du stator (114) prévu dans une cavité interne entourée par la pluralité de dents de stator (112), et les dents de stator sont enroulées de bobines de stator (113) servant d'actionneurs ;
dans lequel l'ensemble rotor (12) comprend une bague de rotor (121) en forme d'anneau circulaire, un aimant d'entraînement du rotor (122) disposé sur une surface inférieure de la bague de rotor (121), et un aimant de poussée du rotor (124) disposé dans une cavité interne de la bague de rotor (121) ;
dans lequel le corps de poussée du stator (114) et l'aimant de poussée du rotor (124) sont configurés pour générer des lignes magnétiques axiales et produire une force de répulsion axiale entre le corps de poussée du stator (114) et l'aimant de poussée du rotor (124) ;
dans lequel l'aimant d'entraînement du rotor (122) comprend une pluralité de parties (123), chaque partie étant magnétisée dans une direction axiale et les parties adjacentes ayant des directions de magnétisation opposées, de sorte que l'aimant d'entraînement du rotor (122) présente une pluralité de pôles magnétiques alternés ; et
dans lequel les bobines de stator (113) sont configurées pour générer : un champ magnétique de composante d'axe direct qui est aligné avec un champ magnétique de chaque partie (123) de l'aimant d'entraînement du rotor (122), pour ajuster une position axiale de l'ensemble rotor (12) ; et un champ magnétique de composante d'axe en quadrature qui est décalé du champ magnétique de chaque partie (123) de l'aimant d'entraînement du rotor (122) d'une demi-longueur d'une partie et n'est donc pas aligné avec le champ magnétique de chaque partie (123) de l'aimant d'entraînement du rotor (122), afin d'entraîner l'ensemble rotor (12) en rotation et de régler une vitesse de rotation de l'ensemble rotor (12).

2. Le moteur à suspension magnétique (10) selon la revendication 1, **caractérisé en ce que** chaque partie (123) de l'aimant d'entraînement du rotor (122) a une forme en arc, de sorte que chaque partie (123) peut être disposée sur la surface inférieure de l'anneau de rotor (121) en suivant la forme de la bague de rotor (121) ; de préférence, l'aimant d'entraînement du rotor (122) comprend quatre parties (123) et l'ensemble stator (11) comprend huit dents de stator (112) ; et de préférence, chacune des parties (123) comprend un ou plusieurs segments magnétiques.

3. Le moteur à suspension magnétique (10) selon la revendication 2, **caractérisé en ce que** les extrémités de la pluralité de parties (123) sont en butée les unes contre les autres, de sorte que l'aimant d'entraînement du rotor (122) constitué de la pluralité de parties (123) présente une forme d'anneau fermé ; ou
dans lequel les extrémités de la pluralité de parties (123) sont espacées les unes des autres, de sorte que des espaces sont présents entre les parties correspondantes.

4. Le moteur à suspension magnétique (10) selon la revendication 1, **caractérisé en ce que** la pluralité de dents de stator (112) est configurée pour s'étendre sensiblement verticalement ou sensiblement en spirale vers le haut depuis la surface supérieure de la base de stator (111) vers l'entrefer (13), de préférence, la pluralité de dents de stator (112) est configurée de telle sorte qu'un espacement entre des dents de stator adjacentes diminue progressivement lorsque les dents de stator (112) s'étendent sensiblement en spirale vers le haut en direction de l'entrefer (13).

5. Le moteur à suspension magnétique (10) selon la revendication 1, **caractérisé en ce que** l'aimant de poussée du rotor (124) et le corps de poussée du stator (114) sont tous deux configurés comme des aimants cylindriques solides, et dans lequel le centre de l'aimant de poussée du rotor (124) et le centre du corps de poussée du stator (114) coïncident tous deux avec l'axe central vertical (A-A) du moteur à suspension magnétique (10) dans un état idéal, de préférence, l'aimant de poussée du rotor (124) a un diamètre extérieur supérieur à celui du corps de poussée du stator (114).

6. Le moteur à suspension magnétique (10) selon la revendication 1, **caractérisé en ce que** l'aimant de poussée du rotor (124) est configuré comme un aimant annulaire comportant une cavité interne et le corps de poussée du stator (114) est configuré comme un aimant cylindrique solide, et dans lequel un centre de l'aimant de poussée du rotor (124) et un centre du corps de poussée du stator (114) coïncident tous deux avec l'axe central vertical (A-A) du moteur à suspension magnétique (10) dans un état idéal ; de préférence, l'aimant de poussée du rotor (124) présente un diamètre interne d'une première taille et un diamètre externe d'une deuxième taille, et le corps de poussée du stator (114) présente un diamètre externe d'une taille comprise entre la première taille et la deuxième taille de l'aimant de poussée du rotor (124).

7. Le moteur à suspension magnétique (10) selon la revendication 1, **caractérisé en ce que** la base du stator (111) a une forme annulaire ou une forme de tarte ronde.

8. Le moteur à suspension magnétique (10) selon la revendication 1, **caractérisé en ce que** le corps de poussée du stator (114) est situé à une position au-dessus des bobines de stator (113) et à proximité des têtes des dents du stator (112), de préférence, le corps de poussée du stator (114) est constitué de matériaux magnétiques permanents ou est formé de bobines électromagnétiques ou d'électroaimants, et de préférence l'aimant de poussée du rotor (124) et l'aimant d'entraînement du rotor (122) sont tous deux constitués de matériaux magnétiques permanents.

9. Le moteur à suspension magnétique (10) selon la revendication 1, **caractérisé en ce que** la base du stator (111) est constituée de matériaux magnétiquement conducteurs pour assurer une connexion magnétiquement conductrice entre les racines de la pluralité de dents de stator (112) ; et/ou
dans lequel la bague de rotor est constituée de matériaux magnétiquement conducteurs pour assurer une connexion magnétiquement conductrice entre les parties correspondantes de l'aimant d'entraînement du rotor (122).

10. Le moteur à suspension magnétique (10) selon la revendication 1, **caractérisé en ce que** chacune des dents de stator (112) est enroulée d'une bobine de stator (113), et chacune des bobines de stator (113) est située entre une racine et une tête d'une dent de stator correspondante (112) et s'étend sur une partie de la longueur de la dent de stator correspondante (112), de préférence, les bobines de stator (113) sont interconnectées en groupes pour former une pluralité de groupes de bobines de stator contrôlables indépendamment, de préférence, chaque groupe de bobines de stator est connecté à un amplificateur pour permettre à des courants de circuler dans les bobines de stator (113) du groupe de bobines de stator, et de préférence, chaque amplificateur est commandable indépendamment pour permettre à des courants d'amplitudes différentes de circuler dans les bobines de stator (113) du groupe de bobines de stator connecté à l'amplificateur.

11. Le moteur à suspension magnétique (10) selon la revendication 10, **caractérisé en ce que** le moteur à suspension magnétique (10) comprend un contrôleur configuré pour contrôler une valeur de courant envoyé à chaque amplificateur, de sorte que le courant dans chaque groupe de bobines de stator soit conforme à une valeur de courant prédéfinie pour ce groupe de bobines de stator, contrôlant ainsi au moins l'un parmi un mouvement axial, un mouvement d'inclinaison et un mouvement de rotation de l'ensemble rotor (12).

12. Le moteur à suspension magnétique (10) selon la revendication 11, **caractérisé en ce que** le contrôleur est configuré pour effectuer au moins l'une des opérations suivantes :
i) le contrôleur ajuste le champ magnétique de la composante d'axe direct en ajustant et en contrôlant la valeur du courant envoyé à l'amplificateur, ajustant et contrôlant ainsi une force axiale générée sur l'ensemble rotor (12) pour garantir que l'entrefer axial (13) entre l'ensemble rotor (12) et l'ensemble stator (11) soit conforme à une valeur de distance prédéfinie ;
ii) le contrôleur ajuste le champ magnétique de composante d'axe en quadrature en ajustant et en contrôlant la valeur du courant envoyé à l'amplificateur, ajustant et contrôlant ainsi un couple de rotation généré sur l'ensemble rotor (12) afin de garantir que la vitesse de rotation de l'ensemble rotor (12) soit conforme à une valeur de vitesse de rotation prédéfinie ; et
iii) le contrôleur ajuste et commande la valeur du courant envoyé aux bobines de stator (113) qui correspond à deux des parties opposées l'une à l'autre, ajustant et commandant ainsi un moment de basculement généré sur l'ensemble rotor (12) afin de garantir qu'un angle de basculement de l'ensemble rotor (12) soit conforme à une valeur d'angle de basculement prédéfinie.

13. Le moteur à suspension magnétique (10) selon la revendication 11, **caractérisé en ce que** le moteur à suspension magnétique (10) comprend un ou plusieurs capteurs destinés à mesurer au moins l'une parmi la position axiale, un angle de basculement et une position angulaire de l'ensemble rotor (12) par rapport à l'ensemble stator (11), de préférence, le contrôleur est configuré pour recevoir les valeurs mesurées provenant du ou des capteurs et ajuster la valeur du courant envoyé au groupe de bobines du stator sur la base des valeurs mesurées afin de mettre en œuvre la commande, et de préférence, les capteurs sont des capteurs à courants de Foucault ou des capteurs à effet Hall.

14. Le moteur à suspension magnétique (10) selon la revendication 1, **caractérisé en ce que** la tête de chaque dent de stator (112) a une taille supérieure à celle des autres parties de la dent de stator (112), de préférence, la tête de la dent de stator (112) est pourvue d'une surface chanfreinée ou inclinée, de telle sorte que la tête de la dent de stator (112) est en forme de coin.

15. Une pompe à sang à suspension magnétique, **caractérisée en ce que** la pompe à sang à suspension magnétique comprend une roue et un moteur à suspension magnétique (10) selon l'une quelconque des revendications 1 à 14, dans laquelle le moteur à suspension magnétique (10) est utilisé pour entraîner la roue.
